(19) Europäisches Patentamt
European Patent Office
Office européen des brevets

(11) EP 1 737 540 B1

(12) **EUROPEAN PATENT SPECIFICATION**

(45) Date of publication and mention of the grant of the patent:
**21.07.2010 Bulletin 2010/29**

(21) Application number: **05727203.1**

(22) Date of filing: **09.03.2005**

(51) Int Cl.:
***A61Q 19/00*** *(2006.01)* ***A61K 8/81*** *(2006.01)*

(86) International application number:
**PCT/US2005/008052**

(87) International publication number:
**WO 2005/087185 (22.09.2005 Gazette 2005/38)**

(54) **PERSONAL CLEANSING COMPOSITIONS**

ZUSAMMENSETZUNGEN ZUR PERSÖNLICHEN REINIGUNG

COMPOSITIONS POUR NETTOYAGE PERSONNEL

(84) Designated Contracting States:
**AT BE BG CH CY CZ DE DK EE ES FI FR GB GR HU IE IS IT LI LT LU MC NL PL PT RO SE SI SK TR**

(30) Priority: **11.03.2004 US 797981**

(43) Date of publication of application:
**03.01.2007 Bulletin 2007/01**

(73) Proprietor: **The Procter and Gamble Company**
**Cincinnati, Ohio 45202 (US)**

(72) Inventors:
- **KUHLMAN, Dennis, Eugene**
  **Middletown, Ohio 45044 (US)**
- **KYTE, Kenneth, Eugene, III**
  **Lebanon, Ohio 45036 (US)**
- **COFFINDAFFER, Timothy, Woodrow**
  **Maineville, Ohio 45039 (US)**
- **MCCLURE, Matthew, Donald**
  **Loveland, Ohio 45140 (US)**

(74) Representative: **Wilding, Richard Alan et al**
**Procter & Gamble**
**Technical Centres Ltd**
**Rusham Park**
**Whitehall Lane**
**Egham, Surrey TW20 9NW (GB)**

(56) References cited:

| | |
|---|---|
| **WO-A-01/17489** | **FR-A- 2 769 838** |
| **US-A- 5 674 509** | **US-A- 6 150 313** |
| **US-A- 6 159 483** | **US-A1- 2002 071 818** |
| **US-A1- 2003 118 540** | **US-A1- 2003 199 593** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 737 540 B1

**Description**

FIELD OF THE INVENTION

The present invention is for lathering, personal cleansing compositions providing superior cleansing with low irritation and good rinsing from skin, hair and the like.

BACKGROUND OF THE INVENTION

Personal cleansing compositions are an important part of the daily hygienic regimen for a large group of both women and men throughout the world. It is, however, understood that certain cleansers, particularly facial cleansers, while good at removing sebum and grime from the skin and hair, may also cause insult in the form of skin irritation and, or damage to the surfaces being cleansed.

Formulators are challenged to reduce the irritation and damage caused by scrubs, while not compromising the formulations' ability to cleanse. To maintain sufficient cleansing action and/or exfoliation such formulations often include particulates, such as beads and encapsulated materials to replace harsh surfactants. Furthermore, in addition to cleansing, formulators are challenged to condition the hair and skin while minimizing damage and irritation. To accomplish this formulators suspend particles and, or droplets of materials such as oils or petrolatum to provide conditioning benefits. Particulates and oils droplets, however, must be suspended in these compositions in order to minimize migration (up or down) in the package they are sold in. While this is most easily accomplished in a thick, non-lathering composition, consumers often desire lathering products due to their spreading and rinsing characteristics while still in a mild surfactant base.

Suspending particulates such as beads, capsules and, or droplets of materials in these types of formulations is frequently accomplished through the use of suspension polymers. Suspension polymers known in the art for this purpose include acrylate cross-linked acid copolymers. Such materials are manufactured and sold by a number of suppliers including Noveon™, Inc. Noveon's sales literature discloses that addition of slight amounts of a polyol, such as glycerin or polyethylene glycol, may help reduce the "graininess" associated with gel compositions due to agglomeration of the polymer and, or compositional post-neutralization steps resulting in formation of salts; see "Polymer For Personal Care, Carbopol EDT® Resins: Formulation Tips", March 1994.

Notwithstanding the benefits prescribed by Noveon™, use of such suspending agents in cleansing compositions can also impact the user's perception of rinsing the cleanser from the skin and hair. Through exhaustive consumer study, it is noted that when using these suspending polymers in cleanser compositions, consumers are left with the perception that the composition's lather poorly and, or do not rinse easily. In fact, it's not unusual for consumers to be left with the impression that these compositions feel slimy and, or do not rinse easily off after use.

It has also been noted that combinations of surfactants are also important for consumer satisfaction. Consumers appreciate surfactant systems that provide ample and rich lather yet are mild to the skin. High levels of amphoteric and/or zwitterionic surfactants relative to anionic surfactants are key for building mildness. High levels of amphoteric and/or zwitterionic surfactants, however, typically result in high levels of salt that negatively impacts the suspension capability of polymeric suspension systems. Absent such, many of these compositions are not commercially viable. Thus, there is a consumer demand for cleansing or scrub conditioning compositions that provide ample lather while being readily rinsed from the hair and skin.

SUMMARY OF THE INVENTION

The present invention is a lathering cleansing composition comprising an alkyl ethoxylated polymer, at least one lathering surfactant, an acrylate cross linked copolymer, and a particulate material as defined in claim 1. These compositions provide good lathering and are readily rinse off. The particulate materials enhance cleansing and exfoliation as well as provide conditioning benefits without damage or irritation.

All percentages disclosed herein, unless otherwise stated, are by weight of the named material itself that is found in the compositions, thereby excluding for example the weight associated with carriers, impurities and by-products found in the raw material.

DETAILED DESCRIPTION OF THE INVENTION

Alkyl Ethoxylated Polymers

The compositions of the present invention comprise alkyl ethoxylated polymers that are selected from the group consisting of di-alkyl, tri-alkyl- and combinations of di-alkyl and tri-alkyl substituted alkyl ethoxylated polymers. Alternatively mono-alkyl, di-alkyl, tri-alkyl, tetra-alkyl and all combinations thereof substituted alkyl ethoxylated polymers. The alkyl group can be saturated or unsaturated, branched or linear and contain a number of carbon atoms from about 12 carbon atoms to about 50 carbon atoms. The number of moles of ethylene oxide is greater than about greater than about 20, alternatively greater than about 40. These polymers are at levels from 0.2% to 1.0% alternatively from 0.1% to 2.0% and alternatively from 0.05% to 5.0%, of the composition.

The alkyl substitution of the alkyl ethoxylated polymer includes mono-alkyl, di-alkyl, tri-alkyl and tetra-alkyl substitution of the polymer and combinations thereof. Examples of the polymers that are mono alkyl substituted include: Steareth-100 available as Brij 700® from Uniqema Inc., Pareth alcohols available as Performathox 480® and 490® available from New Phase Technologies, Inc. The di- alkyl substituted polymers include PEG 120 methyl glucose dioleate available as Glutamate DOE-120® and Glucamate DOE-120® both from Chemron Corporation. The tri- alkyl substituted polymers include PEG 120 methyl glucose trioleate available as Glucamate LT® from Chemron Corporation. The tetra-alkyl substituted polymers include PEG 150 pentaerythrityl tetrastearate available as Crothix® from Croda Corporation. In the present invention, the usual substitution is di-alkyl, tri-alkyl or tetra-alkyl substitution of the polymer and combinations thereof. Even more usual are the di-alkyl, tri-alkyl substitution of the polymer and combinations thereof

Cross-Linked Copolymers

The composition of the present invention includes cross-linked copolymers, are selected from the group consisting of cross-linked acid copolymers, cross-linked maleic anhydride copolymers and combinations thereof.

The cross-linked copolymers of the present invention are those typically used for thickening and suspending cosmetic agents in compositions such as shampoos, lotions and creams or other products having an aqueous electrolyte containing environment. The cross-linked copolymers are present in the present invention at levels from 1% to 2%, alternatively 0.5% to 3% and alternatively from 0.1% to 5% by weight of the composition.

The cross-linked acid copolymers in the present invention include alkyl substituted acid copolymers One class of alkyl substituted copolymers include a rheology modifying copolymer containing a cross-linked copolymer selected from the group consisting of unsaturated carboxylic acid, a hydrophobic monomer, a hydrophobic chain transfer agent, a cross linking agent, a steric stabilizers and combinations thereof. Carbopol EDT 2020™ from Noveon™ is an example of this suspending agent. Details regarding such suspending agents are found in U.S. 6,433,061, Marchant et al., August 13, 2002.

Another class of copolymers includes a substantially cross-linked alkali-swellable acrylate copolymer described in US 6,635,702. Carbopol Aqua SF-1™ from Noveon™ is an example of this type of suspending agent. Another class of commercially available copolymers useful herein include copolymers of $C_{10-30}$ alkyl acrylates with one or more monomers of acrylic acid, methacrylic acid, or one of their short chain (i.e. $C_{1-4}$ alcohol) esters, wherein the cross linking agent is an allyl ether of sucrose or pentaerytritol. These copolymers are known as acrylates/ $C_{10-30}$ alkyl acrylate cross polymers and are commercially available as Carbopol® 1342, Pemulen® TR-1, and Pemulen® TR-2, from Noveon™. Yet another class of copolymers include polymers that fall under the description of acrylates/vinyl alkyl cross polymers, commercially available as Stabylen® 30 from 3V, Inc.

The cross-linked maleic anhydride copolymers include cross linked $C_1$-$C_{10}$ alkyl vinyl ether/maleic anhydride copolymers. Stabileze QM™ from ISP Corporation is an example of this type of material. In order to be effective, the maleic anhydride segment of this copolymer needs to be at least partially neutralized so that the copolymer becomes anionic.

Particularly useful are cross-linked cross copolymers include cross-linked alkyl substituted acid copolymers and alkali-swellable acrylate copolymers.

As described in Noveon's Technical Data Sheet #244, suspending capability of the formula can be approximated by yield value measurements. One method is the Brookfield yield value extrapolation method. The Brookfield Yield Value (herein BYV) is calculated by the following formula:

$$BYV(dyn/cm^2) = \frac{(\eta_1 - \eta_2)}{100}$$

where, $\eta_1$ and $\eta_2$ are the apparent viscosities of the sample, measured at spindle speeds of 0.5 rpm and 1.0 rpm, respectively.

To determine the BYV for compositions having viscosities from about 5,000 cP to about 25,000 cP at 20 RPM, use a Brookfield viscometer with RV2 and RV3 spindles. As known by a person skilled in the art, in order to successfully suspend materials in the compositions, the BYV of such compositions varies depending on relationship of particulate size of the suspended material and the difference in densities of the dispersed phase and the continuous phase as

expressed by Stokes Law. In the present invention, the BYV is greater than about 150 dyn/$cm^2$, alternatively greater than about 100 dyn/$cm^2$, alternatively greater than about 75 dyn/$cm^2$ and alternatively greater than about 50 dyn/$cm^2$.

Particulate Materials

Particulate materials for use in the present invention can generally be generally classified into one of two groups. These groups include: (1) cleaning or exfoliating agents and (2) conditioning agents.

The particulate cleansing or exfoliating agents can be derived from a wide variety of materials including those derived from inorganic, organic, natural, and synthetic sources.

The particulate cleansing or exfoliating agents of the present invention typically comprise from 1% to 5% alternatively from alternatively from 0.5% to 15% and alternatively from 0.1% to 30% by weight of the composition. Non-limiting examples of these materials include those selected from the group consisting of almond meal, alumina, aluminum oxide, aluminum silicate, apricot seed powder, attapulgite, barley flour, bismuth oxychloride, boron nitride, calcium carbonate, calcium phosphate, calcium pyrophosphate, calcium sulfate, cellulose, chalk, chitin, clay, corn cob meal, corn cob powder, corn flour, corn meal, corn starch, diatomaceous earth, dicalcium phosphate, dicalcium phosphate dihydrate, fullers earth, hydrated silica, hydroxyapatite, iron oxide, jojoba seed powder, kaolin, loofah, magnesium trisilicate, mica, microcrystalline cellulose, montmorillonite, oat bran, oat flour, oatmeal, peach pit powder, pecan shell powder, polybutylene, polyethylene, polyisobutylene, polymethylstyrene, polypropylene, polystyrene, polyurethane, nylon, teflon (i.e. polytetrafluoroethylene), polyhalogenated olefins, pumice rice bran, rye flour, sericite, silica, silk, sodium bicarbonate, sodium silicoaluminate, soy flour synthetic hectorite, talc, tin oxide, titanium dioxide, tricalcium phosphate, walnut shell powder, wheat bran, wheat flour, wheat starch, zirconium silicate, and mixtures thereof. Also useful are particles made from mixed polymers (e.g., copolymers, terpolymers, etc.), among such are polyethylene/polypropylene copolymer, polyethylene/pro- pylene/isobutylene copolymer, polyethylene/styrene copolymer, and mixtures thereof. Typically, the polymeric and mixed polymeric particles are treated via an oxidation process to destroy impurities and the like. The polymeric and mixed polymeric particles can also optionally be cross linked with a variety of common crosslinking agents, non-limiting examples including butadiene, divinyl benzene, methylenebisacrylamide, allyl ethers of sucrose, allyl ethers of pentaerythritol, and mixtures thereof. Other examples of useful particles include waxes and resins such as paraffins, camuba wax, ozekerite wax, candellila wax, urea-formaldehyde resins, and the like. When such waxes and resins are used herein it is important that these materials are solids at ambient and skin temperatures.

Among the preferred water-insoluble, particulate materials useful herein are the synthetic polymeric particles and oils. Synthetic polymeric particles useful in the present invention are selected from the group consisting of polybutylene, polyethylene, polyisobutylene, polymethylstyrene, polypropylene, polystyrene, polyurethane, nylon, teflon, and mixtures thereof.

Oils suitable for use herein include any natural and synthetic materials with an overall solubility parameter less than about 12.5 (cal/$cm^3$), preferably less than about 11.5 (cal/$cm^3$).

By "overall solubility parameter" is meant that it is possible to use oils with higher solubility parameters than 12.5 (cal/$cm^3$) if they are blended with other oils to reduce the overall solubility parameter of the oil mixture to less than about 12.5 (cal/$cm^3$). For example, a small portion of diethylene glycol (sol par = 13.61) could be blended with lanolin oil (sol par = 7.3) and a co-solubilizing agent to create a mixture that has a solubility parameter of less than 12.5 cal/$cm^3$.

Solubility parameters for the oils described herein are determined by methods well known in the chemical arts for establishing the relative polar character of a material. A description of solubility parameters and means for determining them are described by C. D. Vaughn, "Solubility Effects in Product, Package, Penetration and Preservation" 103 Cosmetics and Toiletries 47-69, October 1988; and C. D. Vaughn, "Using Solubility Parameters in Cosmetics Formulation", 36 J. Soc. Cosmetic Chemists 319-333, September/October, 1988.

The conditioning particulate materials of the present invention may be present and typically comprise from 2% to 15%, alternatively from 1% to 20% alternatively from alternatively from 0.5% to 30% and alternatively from 0.1% to 50% by weight of the composition. These oils include but are not limited to hydrocarbon oils and waxes, silicones, fatty acid derivatives, cholesterol, cholesterol derivatives, diglycerides, triglycerides, vegetable oils, vegetable oil derivatives, acetoglyceride esters, alkyl esters, alkenyl esters, lanolin and its derivatives, wax esters, beeswax derivatives, sterols and phospholipids, and combinations thereof.

Non-limiting examples of hydrocarbon oils and waxes suitable for use herein include petrolatum, mineral oil, micro-crystalline waxes, polyalkenes, paraffins, cerasin, ozokerite, polyethylene, perhydrosqualene, poly alpha olefins, hydrogenated polyisobutenes and combinations thereof.

Non-limiting examples of silicone oils suitable for use herein include dimethicone copolyol, dimethylpolysiloxane, diethylpolysiloxane, mixed $C_1$-$C_{30}$ alkyl polysiloxanes, phenyl dimethicone, dimethiconol, and combinations thereof. Preferred are non-volatile silicones selected from dimethicone, dimethiconol, mixed $C_1$-$C_{30}$ alkyl polysiloxane, and combinations thereof. Non-limiting examples of silicone oils useful herein are described in U.S. Patent No. 5,011,681 (Ciotti et al.).

Non-limiting examples of diglycerides and triglycerides suitable for use herein include castor oil, soy bean oil, derivatized soybean oils such as maleated soy bean oil, safflower oil, cotton seed oil, corn oil, walnut oil, peanut oil, olive oil, cod liver oil, almond oil, avocado oil, palm oil and sesame oil, vegetable oils, sunflower seed oil, and vegetable oil derivatives; coconut oil and derivatized coconut oil, cottonseed oil and derivatized cottonseed oil, jojoba oil, cocoa butter, and combinations thereof. In addition any of the above oils that have been partially or fully hydrogenated are also suitable.

Non-limiting examples of acetoglyceride esters suitable for use herein include acetylated monoglycerides.

Non-limiting examples of alkyl esters suitable for use herein include isopropyl esters of fatty acids and long chain esters of long chain fatty acids, e.g. SEFA (sucrose esters of fatty acids). Lauryl pyrolidone carboxylic acid, pentaerythritol esters, aromatic mono, di or triesters, and cetyl ricinoleate are non-limiting examples of which include isopropyl palmitate, isopropyl myristate, cetyl ricinoleate and stearyl ricinoleate. Other examples are: hexyl laurate, isohexyl laurate, myristyl myristate, isohexyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, acyl isononanoate lauryl lactate, myristyl lactate, cetyl lactate, and combinations thereof.

Non-limiting examples of alkenyl esters suitable for use herein include oleyl myristate, oleyl stearate, oleyl oleate, and combinations thereof.

Non-limiting examples of lanolin and lanolin derivatives suitable for use herein include lanolin, lanolin oil, lanolin wax, lanolin alcohols, lanolin fatty acids, isopropyl lanolate, acetylated lanolin, acetylated lanolin alcohols, lanolin alcohol linoleate, lanolin alcohol ricinoleate, hydroxylated lanolin, hydrogenated lanolin and combinations thereof.

Still other suitable oils include milk triglycerides (e.g., hydroxylated milk glyceride) and polyol fatty acid polyesters.

Still other suitable oils include wax esters, non-limiting examples of which include beeswax and beeswax derivatives, spermaceti, myristyl myristate, stearyl stearate, and combinations thereof. Also useful are vegetable waxes such as carnauba and candelilla waxes; sterols such as cholesterol, cholesterol fatty acid esters; and phospholipids such as lecithin and derivatives, sphingo lipids, ceramides, glycosphingo lipids, and combinations thereof.

The conditioning agents useful in the present invention are selected from the group consisting of droplets of emollient oils, skin care actives, vitamins, capsules and mixtures thereof. The capsules are generally made of gelatin, agar, or water-insoluble polymers and may contain emollient oils, vitamins, colored pigment, and additional ingredients, such as hair and skin actives as described below. The particle sizes of these capsules range from about 5 to about 3000 microns.

Lathering Surfactants

The articles of the present invention also comprise one or more lathering surfactants. A lathering surfactant defined herein as surfactant, which when combined with water and mechanically agitated generates a foam or lather. Preferably, these surfactants or combinations of surfactants should be mild, which means that these surfactants provide sufficient cleansing or detersive benefits but do not overly dry the skin or hair while still meeting the lathering criteria described above.

A wide variety of lathering surfactants are useful herein and include those selected from the group consisting of anionic lathering surfactants, nonionic lather surfactants, amphoteric lathering surfactants, and mixtures thereof. Generally, the lathering surfactants are fairly water soluble. When used in the composition, at least about 4% of the lathering surfactants have a HLB value greater than about ten. Examples of such surfactants are found in and U.S. Pat. 5,624,666, to Coffindaffer et al., issued April 29, 1997. Cationic surfactants can also be used as optional components, provided they do not negatively impact the overall lathering characteristics of the required lathering surfactants

Concentrations of these surfactant are from about 10% to about 20%, alternatively from about 6% to about 25%, and alternatively from about 4% to about 30% by weight of the composition. To avoid skin irritation issues, the compositions should have a ratio by weight of the composition of anionic surfactant to amphoteric and/or zwitterionic surfactant is from 1.1: 1 to 1:1.5, alternatively from 1.25:1 to 1:2, and alternatively from about 1.5:1 to 1:3.

Anionic lathering surfactants useful in the compositions of the present invention are disclosed in McCutcheon's, Detergents and Emulsifiers, North American edition (1986), published by allured Publishing Corporation; McCutcheon's, Functional Materials, North American Edition (1992); and U.S. Pat. No. 3,929,678, to Laughlin et al., issued December 30, 1975A wide variety of anionic lathering surfactants are useful herein. Non-limiting examples of anionic lathering surfactants include those selected from the group consisting of sarcosinates, sulfates, sulfonates, isethionates, taurates, phosphates, lactylates, glutamates, and mixtures thereof. Amongst the isethionates, the alkoyl isethionates are preferred, and amongst the sulfates, the alkyl and alkyl ether sulfates are preferred. The alkoyl isethionates typically have the formula $RCO-OCH_2CH_2SO_3M$ wherein R is alkyl or alkenyl, branched or linear of from about 10 to about 30 carbon atoms, preferably less than 20 carbon atoms, most preferably less than 18 carbon atoms and M is a water-soluble cation such as ammonium, sodium, potassium and triethanolamine. Non-limiting examples of these isethionates include those

alkoyl isethionates selected from the group consisting of ammonium cocoyl isethionate, sodium cocoyl isethionate, sodium lauroyl isethionate, and mixtures thereof.

The alkyl and alkyl ether sulfates typically have the respective formulas $ROSO_3M$ and $RO(C_2H_4O)_xSO_3M$, wherein R is alkyl or alkenyl, branched or linear of from about 10 to about 30 carbon atoms, preferably less than 20 carbon atoms, most preferably less than 18 carbon atoms, x is from about 1 to about 10, and M is a water-soluble cation such as ammonium, sodium, potassium and triethanolamine. Another suitable class of anionic surfactants are the water-soluble salts of the organic, sulfuric acid reaction products of the general formula:

$$R1\text{--}SO_3\text{--}M$$

wherein R1 is chosen from the group consisting of a straight or branched chain, saturated aliphatic hydrocarbon radical having from about 8 to about 24, preferably about 10 to about 16, carbon atoms; and M is a cation. Still other anionic synthetic surfactants include the class designated as succinamates, olefin sulfonates having about 12 to about 24 carbon atoms, and b-alkyloxy alkane sulfonates. Examples of these materials are sodium lauryl sulfate and ammonium lauryl sulfate.

Other anionic materials useful herein are soaps (i.e., alkali metal salts, e.g., sodium or potassium salts) of fatty acids, typically having from about 8 to about 24 carbon atoms, preferably from about 10 to about 20 carbon atoms. The fatty acids used in making the soaps can be obtained from natural sources such as, for instance, plant or animal-derived glycerides (e.g., palm oil, coconut oil, soybean oil, castor oil, tallow, lard, etc.). The fatty acids can also be synthetically prepared. Soaps are described in more detail in U.S. Pat. No. 4,557,853, cited above.

Other anionic materials include phosphates such as monoalkyl, dialkyl, and trialkylphosphate salts.

Other anionic materials include alkanoyl sarcosinates corresponding to the formula $RCON(CH_3)CH_2CH_2CO_2M$ wherein R is alkyl or alkenyl of about 10 to about 20 carbon atoms, and M is a water-soluble cation such as ammonium, sodium, potassium and alkanolamine (e.g., triethanolamine), preferred examples of which are sodium lauroyl sarcosinate, sodium cocoyl sarcosinate, ammonium lauroyl sarcosinate, and sodium myristoyl sarcosinate. TEA salts of sarcosinates are also useful.

Also useful are taurates which are based on taurine, which is also known as 2-aminoethanesulfonic acid. Especially useful are taurates having carbon chains between $C_8$ and $C_{16}$. Examples of taurates include N-alkyltaurines such as the one prepared by reacting dodecylamine with sodium isethionate according to the teaching of U.S. Pat. No. 2,658,072, which is incorporated herein by reference in its entirety. Further non-limiting examples include ammonium, sodium, potassium and alkanolamine (e.g., triethanolamine) salts of lauroyl methyl taurate, myristoyl methyl taurate, and cocoyl methyl taurate.

Also useful are lactylates, especially those having carbon chains between $C_8$ and $C_{16}$. Non-limiting examples of lactylates include ammonium, sodium, potassium and alkanolamine (e.g., triethanolamine) salts of lauroyl lactylate, cocoyl lactylate, lauroyl lactylate, and caproyl lactylate.

Also useful herein as anionic surfactants are glutamates, especially those having carbon chains between $C_8$ and $C_{16}$. Non-limiting examples of glutamates include ammonium, sodium, potassium and alkanolamine (e.g., triethanolamine) salts of lauroyl glutamate, myristoyl glutamate, and cocoyl glutamate.

Non-limiting examples of preferred anionic lathering surfactants useful herein include those selected from the group consisting of sodium lauryl sulfate, ammonium lauryl sulfate, ammonium laureth sulfate, sodium laureth sulfate, sodium trideceth sulfate, ammonium cetyl sulfate, sodium cetyl sulfate, ammonium cocoyl isethionate, sodium lauroyl isethionate, sodium lauroyl lactylate, triethanolamine lauroyl lactylate, sodium caproyl lactylate, sodium lauroyl sarcosinate, sodium myristoyl sarcosinate, sodium cocoyl sarcosinate, sodium lauroyl methyl taurate, sodium cocoyl methyl taurate, sodium lauroyl glutamate, sodium myristoyl glutamate, and sodium cocoyl glutamate and mixtures thereof.

Suitable amphoteric or zwitterionic detersive surfactants for use in the compositions herein include those which are known for use in hair care or other personal care cleansing. Concentration of such amphoteric detersive surfactants are from about 1% to about 10%, alternatively from about 0.5 % to about 20% by weight of the composition. Non-limiting examples of suitable zwitterionic or amphoteric surfactants are described in U.S. Patents 5,104,646 (Bolich Jr. et al.) and U.S. Patent 5,106,609 (Bolich, Jr. et al.).

Amphoteric detersive surfactants suitable for use in the compositions are well known in the art, and include those surfactants broadly described as derivatives of aliphatic secondary and tertiary amines in which the aliphatic radical can be straight or branched chain and wherein one of the aliphatic substituents contains from about 8 to about 18 carbon atoms and one contains an anionic water solubilizing group such as carboxy, sulfonate, sulfate, phosphate, or phosphonate. Preferred amphoteric detersive surfactants for use in the present invention are selected from the group consisting of cocoamphoacetate, cocoamphodiacetate, lauroamphoacetate, lauroamphodiacetate, and mixtures thereof.

Commercially available amphoteric surfactants include those sold under the trade names Miranol C2M Conc. N.P., Miranol C2M Conc. O.P., Miranol C2M SF, Miranol CM Special, Miranol Ultra (Rhodia, Inc.); Alkateric 2CIB (Alkaril Chemicals); Amphoterge W-2 (Lonza, Inc.); Monateric CDX-38, Monateric CSH-32 (Mona Industries); Rewoteric AM-2C (Rewo Chemical Group); and Schercoteric MS-2 (Scher Chemicals).

Zwitterionic detersive surfactants suitable for use herein include those surfactants broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group such as carboxy, sulfonate, sulfate, phosphate or phosphonate. Preferred zwitterionic detersive surfactants are the betaines and sulfobetaines, e.g., cocoamidopropylbetaine and cocoamidopropylhydroxysultaine.

Nonionic lathering surfactants for use in the compositions of the present invention are disclosed in McCutcheon's, Detergents and Emulsifiers, North American edition (1986), published by allured Publishing Corporation; and McCutcheon's, Functional Materials, North American Edition (1992); both of which are incorporated by reference herein in their entirety. Nonionic lathering surfactants useful herein include those selected from the group consisting of alkyl glucosides, alkyl polyglucosides, polyhydroxy fatty acid amides, alkoxylated fatty acid esters, lathering sucrose esters, amine oxides, and mixtures thereof.

Alkyl glucosides and alkyl polyglucosides are useful herein, and can be broadly defined as condensation articles of long chain alcohols, e.g. $C_{8-30}$ alcohols, with sugars or starches or sugar or starch polymers, i.e., glycosides or polyglycosides. These compounds can be represented by the formula $(S)_n$-O-R wherein S is a sugar moiety such as glucose, fructose, mannose, and galactose; n is an integer of from about 1 to about 1000, and R is a $C_{8-30}$ alkyl group. Examples of long chain alcohols from which the alkyl group can be derived include decyl alcohol, cetyl alcohol, stearyl alcohol, lauryl alcohol, myristyl alcohol, oleyl alcohol, and the like. Preferred examples of these surfactants include those wherein S is a glucose moiety, R is a $C_{8-20}$ alkyl group, and n is an integer of from about 1 to about 9. Commercially available examples of these surfactants include decyl polyglucoside (available as APG 325 CS from Henkel) and lauryl polyglucoside (available as APG 600CS and 625 CS from Henkel). Also useful are sucrose ester surfactants such as sucrose cocoate and sucrose laurate.

Other useful nonionic surfactants include polyhydroxy fatty acid amide surfactants, more specific examples of which include glucosamides, corresponding to the structural formula:

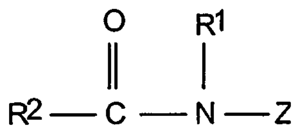

wherein: $R^1$ is H, $C_1$-$C_4$ alkyl, 2-hydroxyethyl, 2-hydroxy- propyl, preferably $C_1$-$C_4$ alkyl, more preferably methyl or ethyl, most preferably methyl; $R^2$ is $C_5$-$C_{31}$ alkyl or alkenyl, preferably $C_7$-$C_{19}$ alkyl or alkenyl, more preferably $C_9$-$C_{17}$ alkyl or alkenyl, most preferably $C_{11}$-$C_{15}$ alkyl or alkenyl; and Z is a polyhydroxy hydrocarbyl moiety having a linear hydrocarbyl chain with a least 3 hydroxyls directly connected to the chain, or an alkoxylated derivative (preferably ethoxylated or propoxylated) thereof. Z preferably is a sugar moiety selected from the group consisting of glucose, fructose, maltose, lactose, galactose, mannose, xylose, and mixtures thereof. An especially preferred surfactant corresponding to the above structure is coconut alkyl N-methyl glucoside amide (i.e., wherein the $R^2CO$- moiety is derived from coconut oil fatty acids). Processes for making compositions containing polyhydroxy fatty acid amides are disclosed, for example, in G.B. Patent Specification 809,060, published February 18, 1959, by Thomas Hedley & Co., Ltd.; U.S. Pat. No. 2,965,576, to E.R. Wilson, issued December 20, 1960; U.S. Pat. No. 2,703,798, to A.M. Schwartz, issued March 8, 1955; and U.S. Pat. No. 1,985,424, to Piggott, issued December 25, 1934; which are incorporated herein by reference in their entirety.

Other examples of nonionic surfactants include amine oxides. Amine oxides correspond to the general formula $R^1R^2R^3NO$, wherein $R^1$ contains an alkyl, alkenyl or monohydroxy alkyl radical of from about 8 to about 18 carbon atoms, from 0 to about 10 ethylene oxide moieties, and from 0 to about 1 glyceryl moiety, and $R^2$ and $R^3$ contain from about 1 to about 3 carbon atoms and from 0 to about 1 hydroxy group, e.g., methyl, ethyl, propyl, hydroxyethyl, or hydroxypropyl radicals. Examples of amine oxides suitable for use in this invention include dimethyldodecylamine oxide, oleyldi(2-hydroxyethyl) amine oxide, dimethyloctylamine oxide, dimethyldecylamine oxide, dimethyl-tetradecylamine oxide, 3,6,9-trioxaheptadecyldiethylamine oxide, di(2-hydroxyethyl)-tetradecylamine oxide, 2-dodecoxyethyldimethylamine oxide, 3-dodecoxy-2-hydroxypropyldi(3-hydroxypropyl)amine oxide, dimethylhexadecylamine oxide.

Non-limiting examples of preferred nonionic surfactants for use herein are those selected form the group consisting of $C_8$-$C_{18}$ glucose amides, $C_8$-$C_{18}$ alkyl polyglucosides, sucrose cocoate, sucrose laurate, lauramine oxide, cocoamine oxide, and mixtures thereof.

Preferred lathering surfactants for use herein are the following, wherein the anionic lathering surfactant is selected from the group consisting of ammonium lauroyl sarcosinate, sodium trideceth sulfate, sodium lauroyl sarcosinate, sodium myristoyl sarcosinate, ammonium laureth sulfate, sodium laureth sulfate, ammonium lauryl sulfate, sodium lauryl

sulfate, ammonium cocoyl isethionate, sodium cocoyl isethionate, sodium lauroyl isethionate, sodium cetyl sulfate, sodium lauroyl lactylate, triethanolamine lauroyl lactylate, and mixtures thereof; wherein the nonionic lathering surfactant is selected from the group consisting of lauramine oxide, cocoamine oxide, decyl polyglucose, lauryl polyglucose, sucrose cocoate, $C_{12-14}$ glucosamides, sucrose laurate, and mixtures thereof; and wherein the amphoteric lathering surfactant is selected from the group consisting of disodium lauroamphodiacetate, sodium lauroamphoacetate, cetyl dimethyl betaine, cocoamidopropyl betaine, cocoamidopropyl hydroxy sultaine, and mixtures thereof.

Lather Volume Test

The Average Lather Volume is a measurement determined by the Lather Volume Test. This test provides a consistent measurement of the volume of lather/foam generated by the articles described herein. The Lather Volume Test protocol is described as follows:

(1) wash hands thoroughly prior to the test using the product being tested. This will remove any soils which may affect the accuracy of the measurement;
(2) towel dry the hands;
(3) dispense, through a syringe, 1cc of the composition in the palm of the hand;
(4) add 2cc of water (medium hardness of about 8-10 grains per gallon) into the hands and rub hands together (very naturally) in a circular motion 5 times;
(5) rotate hand over hand 3 times, collecting the generated lather in a graduated cylinder or beaker big enough to hold the generated lather;
(6) level the lather with a plastic spatula and measure the volume; and
(7) complete the test a total of three times for each composition in order to derive the Average Lather Volume of the composition; calculating the Average Lather Volume by adding the measured lather volumes from each sample and dividing that number by three.

The compositions of the present invention preferably comprise a level of lathering surfactants wherein the Average Lather Volume of the composition is greater than or equal to about 15 ml, alternatively greater than or equal to about 20 ml, even more preferably greater than or equal to about 30 ml.

Additional Ingredients

The compositions of the present invention can contain a wide variety of ingredients including skin and hair care actives that are used in conventional product types, provided that they do not unacceptably alter the benefits of the invention. Additionally, these ingredients, when incorporated into the composition, should be suitable for use in contact with mammalian keratinous tissue without undue toxicity, incompatibility, instability, allergic response, and the like within the scope of sound judgment. The International Cosmetic Ingredient Dictionary and Handbook, 10th Edition (2004) describes a wide variety of non-limiting cosmetic and pharmaceutical ingredients commonly used in the skin care industry, which are suitable for use in the compositions of the present invention. Examples of these and similar ingredient classes include: abrasives, absorbents, aesthetic components such as fragrances, pigments, colorings/colorants, essential oils, skin sensates, astringents, etc. (e.g., clove oil, menthol, camphor, eucalyptus oil, eugenol, menthyl lactate, witch hazel distillate), anti-acne agents (e.g., salicylic acid), anti-wrinkle agents, anti-inflammatory agents, anti-atrophy agents, anti-caking agents, desquamation agents, antimicrobial and antifungal agents (e.g., methylchloroisothiazolinone/ methylisothiazolinone, iodopropynyl butylcarbamate), antioxidants, retinoids, N-acyl amino acid compounds, oil control agents (e.g., dehydroacetic acid or pharmaceutically acceptable salts) binders, biological additives, buffering agents, bulking agents, chelating agents, chemical additives, colorants, cosmetic astringents, cosmetic biocides, denaturants, drug astringents, emollients, external analgesics, film formers or materials, e.g., polymers, for aiding the film-forming properties or substantivity of the composition (e.g., copolymer of eicosene and vinyl pyrrolidone), humectants, opacifying agents, pH adjusters, propellants, reducing agents, sequestering agents, skin bleaching and lightening agents, skin-conditioning agents, skin firming agents, skin soothing and/or healing agents and derivatives, skin treating agents, surfactants, thickeners, amino sugars, and vitamins and derivatives thereof. Additional examples of suitable emulsifiers and surfactants can be found in, for example, U.S. Patent 3,755,560, U.S. Patent 4,421,769, and McCutcheon's Detergents and Emulsifiers, North American Edition, pages 317-324 (1986). It should be noted, however, that many materials may provide more than one benefit, or operate via more than one mode of action. Therefore, classifications herein are made for the sake of convenience and are not intended to limit the active to that particular application or applications listed.

EXAMPLES

Table 1: Examples 1-8

| Ingredients | 1 | 2 | 3 | 4 | 5 | 6 | 7 | 8 |
|---|---|---|---|---|---|---|---|---|
| Acrylates Copolymer[1] | 1.50 | 1.50 | 2.0 | 2.0 | 2.0 | 1.25 | -- | 1.50 |
| Acrylates/$C_{10-30}$ alkyl acrylate crosspolymer[2] | -- | -- | -- | -- | -- | -- | 1.0 | -- |
| Sodium Lauryl Sulfate | 2.0 | -- | -- | -- | -- | -- | -- | -- |
| Sodium Laureth Sulfate | 8.0 | 3.0 | 6.0 | -- | -- | -- | -- | 3.0 |
| Ammonium Lauryl Sulfate | -- | -- | -- | 6.0 | -- | -- | -- | -- |
| Sodium $C_{14-16}$ Olefin Sulfonate | -- | -- | -- | -- | 8.0 | -- | -- | -- |
| Sodium $C_{12-15}$ Pareth-9 Sulfonate | -- | -- | -- | -- | 2.0 | -- | -- | -- |
| Sodium Trideceth Sulfate | -- | -- | -- | -- | -- | 3.0 | 2.5 | -- |
| Sodium Myristoyl Sarcosinate | -- | 5.0 | -- | 2.0 | -- | 3.0 | 2.5 | 5.0 |
| Sodium Lauroamphoacetate[3] | -- | 8.0 | -- | -- | -- | 6.0 | 5.0 | 8.0 |
| Sodium Hydroxide* | pH >6 | pH >6 | pH >6 | -- | pH >6 | -- | -- | pH >6 |
| Triethanolamine* | -- | -- | -- | pH >6 | -- | -- | pH 5.2 | -- |
| Cocamidopropyl Betaine | 4.0 | -- | 8.0 | 7.0 | 10.0 | -- | -- | -- |
| Glycerin | 4.0 | 2.0 | 5.0 | 5.0 | 5.0 | 2.0 | 2.0 | 2.0 |
| Sorbitol | -- | -- | -- | -- | -- | 2.0 | 2.0 | -- |
| Salicylic Acid | -- | -- | -- | -- | 2.0 | 2.0 | 2.0 | -- |
| Fragrance | 0.1 | 0.1 | 0.1 | 0.1 | 0.1 | 0.1 | 0.1 | 0.1 |
| Preservative | 0.3 | 0.3 | 0.3 | 0.3 | 0.3 | 0.15 | 0.15 | 0.3 |
| Jojoba Beads[4] | 1.0 | -- | -- | -- | -- | -- | -- | -- |
| Mica and Titanium Dioxide[5] | -- | 0.2 | -- | -- | 0.2 | -- | -- | 0.2 |
| Mineral Oil, Vitamin E, and Gelatin[6] | -- | -- | 1.0 | -- | -- | -- | -- | -- |
| Pumice[7] | -- | -- | - | 1.0 | -- | -- | -- | -- |
| Oxidized Polyethylene[8] | -- | -- | -- | -- | -- | 2.0 | 2.0 | -- |
| PEG 120 Methyl Glucose Trioleate[9] | 0.5 | -- | -- | -- | 0.5 | 0.25 | 0.25 | -- |
| PEG 120 Methyl Glucose Dioleate[10] | -- | 0.5 | 0.25 | -- | -- | -- | -- | 0.5 |

(continued)

| Ingredients | 1 | 2 | 3 | 4 | 5 | 6 | 7 | 8 |
|---|---|---|---|---|---|---|---|---|
| Petrolatum, USP | -- | -- | -- | -- | -- | -- | -- | 5.0 |
| PEG 150 Pentaerythrityl Tetrastearate[11] | -- | -- | -- | 0.40 | -- | -- | -- | -- |
| Citric Acid** | pH 5.5 | pH 5.5 | pH 5.5 | pH 5.5 | pH 4.5 | pH 5.5 | pH 5.5 | pH 5.5 |
| Water | QS to 100% | QS to 100% | QS to 100% | QS to 100% | QS to 100% | QS to 100% | QS to 100% | QS to 100% |

* per the supplier use directions, the base is used to activate the acrylates copolymer
** acid can be added to adjust the formula to a lower pH
1. Carbopol Aqua SF-1® from Noveon™, Inc.
2. Carbopol Ultrez 21® from Noveon™, Inc.
3. Miranol ® Ultra L32 from Rhodia
4.. Florabeads® from Floratech
5 Pearl 3500® Engelhard
6. Lipopearl® from Lipo Chemicals, Inc.
7. AEC Pumice® from A & E Connock
8. A-C® Polyethylene from Honeywell / Accuscrub® by Accutech
9. Glucamate LT® from Chemron
10. Glucamate DOE-120® from Chemron
11. Crothix® from Croda

Method of making Examples 1-8:

Add Carbopol® to de-ionized free water of the formulation. Add all surfactants except cationics and betaines. If the pH is less than 6 then add a neutralizing agent (typically a base i.e., Triethanolamine, sodium hydroxide) to adjust to a pH greater than 6. If necessary, apply gentle heat to reduce viscosity and help minimize air entrapment. Add betaine and/or cationic surfactants. Add conditioning agents, additional rheology modifiers, pearlizing agents, encapsulated materials, exfoliants, preservatives, dyes, fragrances and other desirable ingredients. Lastly, if desired reduce the pH with an acid (i.e. citric acid) and increase viscosity by adding sodium chloride.

The methods of using the composition of the present invention include cleansing as well as cleansing and conditioning skin and, or hair.

## Claims

**1.** A lathering personal cleansing composition comprising:

a. from 0.06% to 5.0%, preferably from 0.1% to 2.0% of an alkyl ethoxylated polymer, wherein the alkyl substitution preferably comprises mono-alkyl, di-alkyl, tri-alkyl, tetra-alkyl or combinations thereof, more preferably wherein the alkyl substitution comprises di-alkyl, tri-alkyl-or combinations of di-alkyl and tri-alkyl substituted alkyl ethoxylated polymers;
b. from 0.6% to 6% of a cross-linked acid copolymer, preferably wherein the cross linked copolymer comprises cross-linked acid copolymers, cross-linked maleic anhydride copolymers and mixtures thereof;
c. from 0.1% to 50% of a particulate material, wherein the particulate materials comprise cleaning agents, exfoliating agents, or mixtures thereof, derived from inorganic, organic, natural, or synthetic sources; and
d. from 4% to 30%, preferably from 6% to 26% by weight of the composition of a lathering surfactant, comprising anionic lathering surfactants, amphoteric lathering surfactants, and zwitterionic lathering surfactants or mixtures thereof.

**2.** The composition according to claim 1, wherein the alkyl group of the alkyl ethoxylated polymer comprises saturated, unsaturated, branched, linear alkyl groups having from 12 to 50 carbon atoms or combinations thereof.

3. The composition according to claim 1 or 2, wherein the number of moles of ethylene oxide in said alkyl ethoxylated polymer is greater than 40.

4. The composition according to any one of claims 1 to 3, wherein the cross-linked acid copolymer is an alkyle substituted copolymer containing a cross-linked copolymer comprising unsaturated carboxylic acid, a hydrophobic monomer, a hydrophobic chain transfer agent, a cross linking agent, steric stabilizers and combinations thereof.

5. The composition according to any one of claims 1 to 8, wherein the cross-linked copolymers are alkali-swellable acrylate copolymers.

6. The composition according to any one of the preceding claims, further comprising a particulate conditioning agent, wherein the conditioning agent is at a level from 0. 1% to 60% by weight of the composition and preferably comprises droplets of emollient oils, skin care actives, vitamins, capsules containing these materials or mixtures thereof.

7. The composition according to claim 6, wherein the capsules are in the range of 5 to 8000 microns

8. The composition according to any one of the preceding claims comprising lathering surfactant at a level wherein the Average Lather Volume of the composition is greater than or equal to 15 ml.

9. The composition according to any one of the preceding claims, wherein the anionic lathering surfactants comprise sarcosinates, sulfates, sulfonates, isethionates, taurates, phosphates, lactylates, glutamates, or mixtures thereof.

10. The composition according to any one of claims 1 to 9, having a ratio by weight of anionic surfactant to amphoteric and / or zwitterionic surfactants of from 1.6 to 1:3.

11. A method for non-therapeutic cleansing of skin, the method comprising the step of washing the surface area of the skin to be cleansed using a composition according to any one of claims 1 to 10.

## Patentansprüche

1. Schäumende Körperreinigungszusammensetzung, umfassend:

a. von 0,05 % bis 5,0 %, vorzugsweise von 0,1 % bis 2,0 % ein alkylethoxyliertes Polymer, wobei die Alkylsubstitution vorzugsweise Monoalkyl, Dialkyl, Trialkyl, Tetraalkyl oder Kombinationen davon umfasst, wobei mehr bevorzugt die Alkylsubstitution Dialkyl, Trialkyl oder Kombinationen von dialkyl- und trialkyl-substituierten alkylethoxylierten Polymeren umfasst;
b. von 0,5 % bis 5 % ein vernetztes Säurecopolymer, wobei vorzugsweise das vernetzte Copolymer vernetzte Säurecopolymere, vernetzte Maleinsäureanhydrid-Copolymere und Mischungen davon umfasst;
c. von 0,1 % bis 50 % ein teilchenförmiges Material, wobei die teilchenförmigen Materialien Reinigungsmittel, Peelingmittel oder Mischungen davon umfassen, die von anorganischen, organischen, natürlichen oder synthetischen Quellen abgeleitet sind; und
d. von 4 Gew.-% bis 30 Gew.-%, vorzugsweise von 5 Gew.-% bis 25 Gew.-% der Zusammensetzung ein schaumerzeugendes Tensid, das anionische schaumerzeugende Tenside, amphotere schaumerzeugende Tenside und zwitterionische schaumerzeugende Tenside oder Mischungen davon umfasst.

2. Zusammensetzung nach Anspruch 1, wobei die Alkylgruppe des alkylethoxylierten Polymers gesättigte, ungesättigte, verzweigte, lineare Alkylgruppen mit 12 bis 50 Kohlenstoffatomen oder Kombinationen davon umfasst.

3. Zusammensetzung nach Anspruch 1 oder 2, wobei die Molanzahl von Ethylenoxid in dem alkylethoxylierten Polymer mehr als 40 beträgt.

4. Zusammensetzung nach einem der Ansprüche 1 bis 3, wobei das vernetzte Säurecopolymer ein alkylsubstituiertes Copolymer ist, das ein vernetztes Copolymer enthält, das ungesättigte Carbonsäure, ein hydrophobes Monomer, ein hydrophobes Kettenübertragungsmittel, ein Vernetzungsmittel, sterische Stabilisierungsmittel und Kombinationen davon umfasst.

5. Zusammensetzung nach einem der Ansprüche 1 bis 3, wobei die vernetzten Copolymere mit Alkali quellbare Acrylatcopolymere sind.

6. Zusammensetzung nach einem der vorstehenden Ansprüche, die ferner ein teilchenförmiges Konditioniermittel umfasst, wobei das Konditioniermittel in einer Konzentration von 0,1 Gew.-% bis 50 Gew.-% der Zusammensetzung vorliegt, und vorzugsweise Tröpfchen von weichmachenden Ölen, Hautpflege-Wirkstoffe, Vitamine, Kapseln, die diese Materialien enthalten, oder Mischungen davon umfassen.

7. Zusammensetzung nach Anspruch 6, wobei die Kapseln im Bereich von 5 bis 3000 Mikrometer liegen.

8. Zusammensetzung nach einem der vorstehenden Ansprüche, umfassend schaumerzeugendes Tensid in einer Konzentration, wobei das durchschnittliche Schaumvolumen der Zusammensetzung größer als oder gleich 15 ml ist.

9. Zusammensetzung nach einem der vorstehenden Ansprüche, wobei die anionischen schaumerzeugenden Tenside Sarcosinate, Sulfate, Sulfonate, Isethionate, Taurate, Phosphate, Lactylate, Glutamate oder Mischungen davon umfassen.

10. Zusammensetzung nach einem der Ansprüche 1 bis 9, die ein Gewichtsverhältnis von anionischem Tensid zu amphoteren und/oder zwitterionischen Tensiden von 1,5:1 bis 1:3 aufweist.

11. Verfahren zum nichttherapeutischen Reinigen von Haut, wobei das Verfahren den Schritt des Waschens der zu reinigenden Hautoberfläche mit einer Zusammensetzung nach einem der Ansprüche 1 bis 10 umfasst.

## Revendications

1. Composition moussante pour la toilette comprenant :

   a. de 0,05 % à 5,0 %, de préférence de 0,1 % à 2,0 % d'un polymère alkyl éthoxylé, dans laquelle la substitution par un alkyle comprend de préférence un mono-alkyle, un di-alkyle, un tri-alkyle, un tétra-alkyle ou leurs combinaisons, plus préférablement dans laquelle la substitution par un alkyle comprend un di-alkyle, un tri-alkyle- ou des combinaisons de polymères alkyl éthoxylés à substitution di-alkyle et tri-alkyle ;
   b. de 0,5 % à 5 % d'un copolymère acide réticulé, de préférence dans laquelle le copolymère réticulé comprend copolymères acides réticulés, des copolymères d'anhydride maléique réticulés et leurs mélanges ;
   c. de 0,1 % à 30 % d'un matériau particulaire, dans laquelle les matériaux particulaires comprennent des agents de nettoyage, des agents exfoliants, ou leurs mélanges, dérivés de sources inorganiques, organiques, naturelles ou synthétiques ; et
   d. de 4 % à 30 %, de préférence de 6 % à 25 % en poids de la composition d'un agent tensioactif moussant, comprenant des agents tensioactifs moussants anioniques, des agents tensioactifs moussants amphotères, et des agents tensioactifs moussants zwittérioniques ou leurs mélanges.

2. Composition selon la revendication 1, dans laquelle le groupe alkyle du polymère alkyl éthoxylé comprend des groupes alkyle saturés, insaturés, ramifiés, linéaires ayant de 12 à 50 atomes de carbone ou leurs combinaisons.

3. Composition selon la revendication 1 ou 2, dans laquelle le nombre de moles d'oxyde d'éthylène dans ledit polymère alkyl éthoxylé est supérieur à 40.

4. Composition selon l'une quelconque des revendications 1 à 3, dans laquelle le copolymère acide réticulé est un copolymère à substitution alkyle contenant un copolymère réticulé comprenant un acide carboxylique insaturé, un monomère hydrophobe, un agent de transfert de chaîne hydrophobe, un agent de réticulation, des agents stabilisants stériques et leurs combinaisons.

5. Composition selon l'une quelconque des revendications 1 à 3, dans laquelle les copolymères réticulés sont des copolymères acrylate pouvant gonfler dans un alcali.

6. Composition selon l'une quelconque des revendications précédentes, comprenant en outre un agent de conditionnement particulaire, dans laquelle l'agent de conditionnement est à un taux allant de 0,1 % à 50 % en poids de la composition, et comprend de préférence des gouttelettes d'huiles d'émollient, des principes actifs pour le soin de la peau, des vitamines, des gélules contenant ces matériaux ou leurs mélanges.

7. Composition selon la revendication 6, dans laquelle les gélules sont dans l'intervalle de 5 à 3000 microns.

8. Composition selon l'une quelconque des revendications précédentes comprenant un agent tensioactif moussant à un taux auquel le volume moyen de moussage de la composition est supérieur ou égal à 15 mL.

9. Composition selon l'une quelconque des revendications précédentes, dans laquelle les agents tensioactifs moussants anioniques comprennent des sarcosinates, des sulfates, des sulfonates, des iséthionates, des taurates, des phosphates, des lactylates, des glutamates, ou leurs mélanges.

10. Composition selon l'une quelconque des revendications 1 à 9, ayant un rapport en poids d'agent tensioactif anionique sur agents tensioactifs amphotères et / ou zwittérioniques allant de 1,5:1 à 1:3.

11. Procédé pour le nettoyage non thérapeutique de la peau, le procédé comprenant l'étape consistant à laver la surface de la peau à nettoyer en utilisant une composition selon l'une quelconque des revendications 1 à 10.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6433061 B, Marchant
- US 6635702 B
- US 5011681 A, Ciotti
- US 5624666 A, Coffindaffer
- US 3929678 A, Laughlin
- US 4557853 A
- US 2658072 A
- US 5104646 A, Bolich Jr.
- US 5106609 A, Bolich, Jr.
- US 2965576 A
- US 2703798 A
- US 1985424 A
- US 3755560 A
- US 4421769 A


### Non-patent literature cited in the description


- **C. D. Vaughn.** Solubility Effects in Product, Package, Penetration and Preservation. *Cosmetics and Toiletries,* October 1988, vol. 103, 47-69
- **C. D. Vaughn.** Using Solubility Parameters in Cosmetics Formulation. *J. Soc. Cosmetic Chemists,* September 1988, vol. 36, 319-333
- McCutcheon's, Detergents and Emulsifiers. Publishing Corporation, 1986
- McCutcheon's, Functional Materials. 1992
- The International Cosmetic Ingredient Dictionary and Handbook. 2004
- McCutcheon's Detergents and Emulsifiers. 1986, 317-324